# FASCICULE DE BREVET EUROPEEN

(11) **EP 0 824 097 B1**
(45) Date de publication et mention de la délivrance du brevet: **30.12.1998**
(21) Numéro de dépôt: 97401736.0
(22) Date de dépôt: 18.07.1997
(51) Int. Cl.: C07C 271/16, A61K 7/48, A61K 7/06

(54) **Dérivés n-(alkyloxycarbonyl)-N-(2-hydroxy 3-alkyloxypropyl)-éthanolamine et leur utilisation dans et pour la préparation de compositions cosmétiques ou dermatologiques**
N-(Alkyloxycarbonyl)-N-(2-Hydroxy-3-Alkyloxypropyl)-Ethanolamin-Derivate und ihre Verwendung zur Herstellung von kosmetischen oder dermatologischen Zusammensetzungen
N-(Alkyloxycarbonyl)-N-(2-hydroxy-3-alkyloxypropyl)-ethanolamine derivatives and their use for the preparation of cosmetic or dermatologic compositions

(30) Priorité: 14.08.1996 FR 9610230
(43) Date de publication de la demande: 18.02.1998
(73) Titulaire: L'OREAL, 75008 Paris (FR)
(72) Inventeur: Philippe, Michel, 91320 Wissous (FR)
(74) Mandataire: Dodin, Catherine

(56) Documents cités:
- EP-A- 0 227 994
- EP-A- 0 482 860
- EP-A- 0 666 251

## Description

L'invention concerne des dérivés N-(alkyloxycarbonyl)-N-(2-hydroxy-3-alkyloxypropyl)-éthanolamine ainsi que leur utilisation dans et pour la préparation de compositions cosmétiques ou dermatologiques.

L'exposition de la peau au froid, au soleil, aux atmosphères à faible humidité relative, les traitements répétés avec des compositions de lavage ou encore le contact de la peau avec des solvants organiques, sont des facteurs qui entraînent, à des degrés divers, un dessèchement apparent. La peau apparaît plus sèche, moins souple et le relief cutané plus prononcé. Les cheveux peuvent également, après avoir été soumis à de fréquents traitements capillaires, perdre leur aspect brillant et devenir rêches et cassants.
Il s'est donc avéré souhaitable de pouvoir appliquer sur la peau et/ou sur les cheveux des composés permettant de prévenir ou de corriger ces phénomènes et redonner ainsi à la peau sa souplesse et aux cheveux leur brillance et leur douceur.
Pour résoudre ces problèmes, on a déjà proposé d'utiliser dans des compositions cosmétiques ou dermatologiques des amides lipophiles notamment dans les demandes de brevet EP-A-227 994, EP-A-482 860 et DE 43 26 959.

La Demanderesse a découvert de manière surprenante que des dérivés N-(alkyloxy-carbonyl)-N-(2-hydroxy-3-alkyloxypropyl)-éthanolamine particuliers dont on définira la structure ci-dessous présentaient des propriétés cosmétiques intéressantes, en particulier des propriétés hydratantes de la peau.
On a en effet constaté qu'en appliquant ces produits sur la peau, il est possible d'augmenter la fixation d'eau dans le Stratum Corneum. Ces composés peuvent donc être utilisés comme agents hydratants, notamment de la peau chez l'humain.
Ils permettent de conserver ou de restaurer la souplesse de la peau, son élasticité, sa résistance au mouvement du corps et sa fonction de barrière à l'entrée des substances toxiques. Ils peuvent ainsi être utilisés dans des produits de soin pour les peaux sèches ou prédisposés à l'assèchement.
Ces composés présentent de façon inattendue une activité de fixation de l'eau dans le Stratum Cornéum sensiblement plus importante que les amides lipophiles connus en cosmétique pour leurs propriétés hydratantes.

La demanderesse a découvert également de façon surprenante que les dérivés N-(alkyloxycarbonyl)-N-(2-hydroxy-3-alkyloxypropyl)-éthanolamine particuliers selon l'invention, utilisés dans des compositions capillaires, confèrent aux cheveux un toucher particulièrement doux et facilitent leur démêlage. Ils peuvent être utilisés comme agents de conditionnement des cheveux. Un objet de l'invention concerne des dérivés N-(alkyloxycarbonyl)-N-(2-hydroxy-3-alkyloxypropyl)-éthanolamine particuliers dont on indiquera la structure ci-dessous.
Un autre objet de l'invention concerne l'utilisation desdits dérivés N-(alkyloxycarbonyl)-N-(2-hydroxy-3-alkyloxypropyl)-éthanolamine que l'on définira par la suite, dans et pour la préparation de compositions cosmétiques ou dermatologiques.
Un autre objet de l'invention concerne les compositions cosmétiques ou dermatologiques contenant ces dérivés N-(alkyloxycarbonyl)-N-(2-hydroxy-3-alkyloxypropyl)-éthanolamine.
Un autre objet de l'invention concerne donc l'utilisation de ces dérivés comme agents hydratants, dans et pour la préparation de compositions pour le soin de la peau.
Un autre objet de l'invention concerne l'utilisation de ces dérivés N-(alkyloxycarbonyl)-N-(2-hydroxy-3-alkyloxypropyl)-éthanolamine, comme agents de conditionnement des cheveux dans et pour la préparation de compositions capillaires.

Les dérivés N-(alkyloxycarbonyl)-N-(2-hydroxy-3-alkyloxypropyl)-éthanolamine conformes à l'invention répondent à la formule générale (I) suivante: 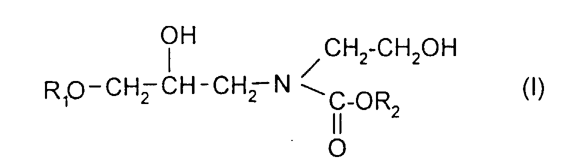 dans laquelle R₁ et R₂, identiques ou différents, représentent un radical alcoyle ayant de 8 à 26 atomes carbone, linéaire ou ramifié, saturé ou insaturé et éventuellement hydroxylé.

Parmi les composés préférés correspondant à la formule générale (I), on peut notamment citer :
- la N-(hexadécyloxycarbonyl)-N-(2-hydroxy-3-hexadécyloxypropyl)-éthanolamine
- la N-(dodécyloxycarbonyl)-N-(2-hydroxy-3-hexadécyloxypropyl)-éthanolamine
- la N-(2-éthyl-hexyloxycarbonyl)-N-(2-hydroxy-3-hexadécyloxypropyl)-éthanol-amine

Ces composés peuvent être obtenus par alkyloxycarbonylation de la fonction amine d'un composé de formule (II) suivante : 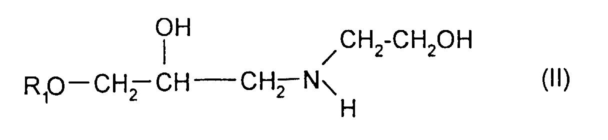 dans laquelle R₁ a les mêmes significations indiquées ci-dessus, en faisant réagir dans un solvant organique (de préférence le tétrahydrofurane), ce composé avec un composé de formule (III) suivante : 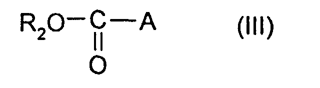 dans laquelle:
- A est un groupe activant choisi parmi un atome d'halogène tel que chlore et un azolide comme le résidu imidazolide de structure : 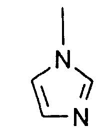
- R₂ a les mêmes significations indiquées ci-dessus puis à ajouter au milieu réactionnel une base organique telle que la triéthylamine.

La synthèse des composés de l'invention est généralement mise en oeuvre selon des méthodes conventionnelles telles que celles décrites dans le document « Advanced Organic Chemistry » édité par J. MARCH.

Les composés de départ de formule (II) sont connus et décrits notamment dans le « Polish Journal of Chemistry, 52, 1283 et dans la demande EP-A-227 994.

Les compositions cosmétiques ou dermatologiques selon l'invention sont caractérisées par le fait qu'elles contiennent dans un véhicule cosmétiquement ou dermatologiquement acceptable au moins un composé de formule (I) tel que défini ci-dessus.
Les compositions cosmétiques ou dermatologiques selon l'invention comprennent de préférence 0,001 à 15% en poids de composé de formule (I) par rapport au poids total de la composition.
Le véhicule cosmétiquement acceptable utilisé dans les compositions de l'invention est choisi parmi l'eau; les solvants organiques compatibles avec une application cutanée ou capillaire tels que l'acétone, l'isopropanol, l'éthanol; les triglycérides d'acides gras à 6-24 atomes de carbone, les éthers de glycol, les esters de polyalkylèneglycols et les silicones volatiles ou leurs mélanges.

Les compositions peuvent se présenter sous forme de lotion aqueuse ou hydroalcoolique, monophasique ou polyphasique, de gel monophasique ou polyphasique, d'émulsion, de crème, de dispersion vésiculaire de lipides ioniques ou non ioniques, lesdites vésicules pouvant alors servir en tant qu'agent d'encapsulation pour des ingrédients actifs lipophiles ou hydrophiles, de mousse, de spray.

Les compositions pour le soin de la peau selon l'invention peuvent se présenter sous forme de lotion, de gel, d'émulsion, de crème ou de mousse à appliquer sur la peau.
Les compositions capillaires peuvent se présenter sous forme de shampooing, d'après-shampooing à rincer ou non, de compositions pour permanente, défrisage, coloration ou décoloration, ou encore sous forme de compositions à rincer, à appliquer avant ou après une coloration, une permanente ou un défrisage ou encore entre les deux étapes d'une permanente ou d'un défrisage.

Les compositions cosmétiques ou dermatologiques peuvent par ailleurs contenir des additifs cosmétiques conventionnels choisis parmi les corps gras, les solvants organiques, les silicones, les épaississants, les adoucissants, les tensio-actifs, les polymères anioniques, cationiques, non-ioniques ou amphotères, les agents anti-mousses, les agents conditionneurs du cheveu tels que des protéines, des vitamines, les agents traitants (agents anti-chute, antipelliculaires), les colorants, les parfums, les conservateurs, les agents propulseurs.

Plus précisément, comme corps gras, on peut utiliser une huile ou une cire ou leur mélange, des acides gras, des alcools gras, des esters d'acides gras tels que les triglycérides d'acide gras en C₆ à C₁₈, de la vaseline, de la paraffine, de la lanoline, de la lanoline hydrogénée ou acétylée.
Parmi les huiles, on peut citer les huiles minérales, animales, végétales ou les huiles de synthèse, et notamment l'huile de vaseline, de paraffine, de ricin, de jojoba, de sésame, ainsi que les huiles et les gommes de silicone et les isoparaffines.
Parmi les cires, on peut citer les cires animales, végétales, minérales ou de synthèse, et notamment les cires d'abeilles, de Candellila, les ozokérites, les cires microcristallines ainsi que les cires et résines de silicone.

Parmi les solvants organiques usuellement utilisés dans les compositions cosmétiques, on peut citer plus précisément les monoalcools ou polyalcools inférieurs en C₁ à C₆ comme l'éthanol, l'isopropanol, l'éthylèneglycol, le diéthylènegycol, le propylèneglycol, le glycérol.
Les agents épaississants peuvent être choisis notamment parmi l'alginate de sodium, la gomme arabique, les dérivés cellulosiques tels que la méthylcellulose, l'hydroxyméthylcellulose, l'hydroxyéthylcellulose, l'hydroxypropylcellulose, l'hydroxypropylméthylcellulose, la gomme de guar ou ses dérivés, la gomme de xanthane, les scléroglucanes, les acides polyacryliques réticulés.
Comme agents tensio-actifs et comme polymères, on peut utiliser tous ceux bien connus de l'état de la technique notamment pour leur utilisation dans des compositions capillaires.

Les compositions peuvent se présenter sous forme de dispersion vésiculaire de lipides amphiphiles ioniques ou non-ioniques. Elles sont préparées notamment en faisant gonfler les lipides dans une solution aqueuse pour former des sphérules dispersées dans le milieu aqueux comme décrit dans STANDISH & WATKINS, J. Mol. Biol., 13,238 (1965) ou dans les brevets FR-A-2.315.991 et FR-A-2.416.008 de la demanderesse. Les différents types de procédés de préparation sont décrits dans "Les liposomes en biologie cellulaire et pharmacologie", Edition INSERM/John Libery Eurotext, 1987, pages 6 à 18.

Le pH des compositions selon l'invention est généralement compris entre 4 et 8 et de préférence entre 5 et 7.

On va maintenant donner à titre d'illustration et sans aucun caractère limitatif plusieurs exemples de préparation de composés selon l'invention ainsi que des exemples de compositions cosmétiques les contenant.

### Exemple 1 : Synthèse de la N-(hexadécyloxycarbonyl)-N-(2-hydroxy-3-hexadécyloxypropyl)-éthanolamine de formule (I) (R₁ = C₁₆ H₃₃, R₂ = C₁₆ H₃₃)

37 g (10,3 x 10⁻² mole) de N-(2-hydroxypropyl-3-hexadécyloxy)-éthanolamine sont mis en solution dans 400 ml de tétrahydrofurane tiède. On ajoute goutte à goutte 31,4 g (10,3 x 10⁻² mole) de chloroformiate de cétyle dans 100 ml de tétrahydrofuranne. La réaction est légèrement exothermique.
Puis on coule 15 ml (10,3 x 10⁻² mole) de triéthylamine. Le milieu hétérogène est chauffé 30 min à 50°C. On vérifie l'absence d'amine par C.C.M.
Après retour à température ambiante, on ajoute 500 ml d'eau. Le précipité formé est filtré, lavé avec de l'eau puis séché.

On obtient 63,6 g de solide blanc (rendement = 98%)

Analyse élémentaire:

| | **C** | **H** | **N** | **O** |
|---|---|---|---|---|
| Calculée (%) | 72,67 | 12,36 | 2,23 | 12,74 |
| Trouvée (%) | 72,71 | 12,40 | 2,24 | 12,89 |

- C.C.M. (éluant = dichlorométhane/méthanol ; 9/1): tache unique
- Rf égal à environ 0,5
- Spectre RMN 1H 200 MHz conforme
- Point de fusion (capillaire) : 72-73°C

### Exemple 2 : Synthèse de la N-(dodécyloxycarbonyl)-N-(2-hydroxy-3-hexadécyloxypropyl)-éthanolamine de formule (I) (R₁ = C₁₆H₃₃; R₂ = C₁₂ H₂₅)

50 g (13,9 x 10⁻² mole) de N-(2-hydroxypropyl-3-hexadécyloxy)-éthanolamine sont mis en solution dans 500 ml de tétrahydrofurane tiède. On ajoute goutte à goutte 34,6 g (13,9 x 10⁻² mole) de chloroformiate de dodécyle dans 100 ml de tétrahydrofuranne. La température atteint 45°C.
On ajoute 19,3 ml (13,9 x 10⁻² mole) de triéthylamine. Le milieu hétérogène est chauffé 30 mn à 50°C. On vérifie l'absence d'amine par C.C.M. Après retour à température ambiante, on ajoute 500 ml d'eau, on refroidit puis on ajoute 400ml d'acétone. Le précipité formé est filtré, lavé avec de l'acétone refroidie à 5°C.

On obtient 71,5 g de solide blanc (rendement = 74%). On recristallise dans l'acétate d'éthyle et on obtient 59,1 g de cristaux blancs.

Analyse élémentaire:

| | **C** | **H** | **N** | **O** |
|---|---|---|---|---|
| Calculée (%) | 71,40 | 12,16 | 2,46 | 14,12 |
| Trouvée (%) | 71,39 | 12,24 | 2,26 | 14,12 |

- C.C.M. (éluant = dichlorométhane/méthanol ; 9/1): tache unique
- Rf égal à environ 0,5
- Spectre RMN 1H 200 MHz conforme
- Point de fusion (capillaire) : 55-56°C

### Exemple 3 : Synthèse de la N-(2-éthyl-hexyloxycarbonyl)-N-(2-hydroxy-3-hexadécyloxypropyl)-éthanolamine de formule (I) (R1 = C₁₆H₃₃, R₂ = CH₃-(CH₂)₃-CH(C₂H₅)-CH₂.)

50 g (13,9 x 10⁻² mole) de N-(2-hydroxypropyl-3-hexadécyloxy)-éthanolamine sont mis en solution dans 500 ml de tétrahydrofurane tiède.
On ajoute goutte à goutte 26,7 g (13,9 x 10⁻² mole) de chloroformiate d'éthyl-2 hexyle. La réaction est légèrement exothermique. Puis on ajoute 19,3 ml (13,9 x 10⁻² mole) de triéthylamine. Le milieu hétérogène est chauffé 30 min à 50°C. On vérifie l'absence d'amine par C.C.M. Après retour à température ambiante, on ajoute 500 ml d'eau et on extrait avec 800ml de dichlorométhane. La phase organique est lavée avec une solution de chlorure de sodium. On sèche la phase organique avec du sulfate de sodium et on évapore à sec. Le liquide obtenu est purifié par chromatographie.

On obtient 62,6 g de liquide incolore (rendement = 84%)

Analyse élémentaire:

| | **C** | **H** | **N** | **O** |
|---|---|---|---|---|
| Calculée (%) | 69,86 | 11,92 | 2,72 | 15,51 |
| Trouvée (%) | 69,20 | 11,85 | 3,04-3,20 | 15,53-15,80 |

- C.C.M. (éluant = dichlorométhane/méthanol ; 9/1): tache unique
- Rf égal à environ 0,5
- Spectre RMN 1H 200 MHz conforme

### Exemple 4: Exemple comparatif

### Mesure de la conductivité de la peau

Cette mesure permet de mettre en évidence la variation du taux d'hydratation de la peau. Elle est effectuée à l'aide d'un appareil comportant une électrode centrale sous forme de tige entourée par une électrode cylindrique. L'appareil est appliqué sur la peau et l'on applique un courant alternatif de haute fréquence. On constate que plus l'hydratation de la peau est importante, plus la quantité de courant consommé est élevée. On met ainsi en évidence l'augmentation de la conductivité de la peau et donc l'augmentation de son taux d'hydratation.

Cette mesure est effectuée pour :
a) la N-(dodécyloxycarbonyl)-N-(2-hydroxy-3-hexadécyloxypropyl)-éthanolamine (composé de l'exemple 2) ;
b) la N-dodécanoyl-N-(2-hydroxy-3-hexadécyloxypropyl)-éthanolamine, homologue amide connu dans la demande EP-A-227994 pour être un agent hydratant que l'on nommera reférence A ;

Le composé de l'exemple 2 et celui de référence A sont préparés à raison de 3% dans le mélange dichlorométhane/méthanol (2/1 en volume) puis appliqués sur des morceaux de stratum corneum délipidé.

On mesure, 20 heures après l'application, la conductivité du stratum corneum pour chaque composé.

On obtient les résultats suivants

| Composé | Example 2 | Référence A |
|---|---|---|
| Conductivité | +44 +/-14 | -14 +/-8 |

On constate que la conductivité de la peau est augmentée lorsqu'elle est traitée avec le composé selon l'invention contrairement avec le composé de référence A. Ce composé permet donc bien d'en augmenter le taux d'hydratation.

### Exemple 5 : Shampooing

- Alkyl (C₉/C₁₀/C₁₁ - 20/40/40) polyglucoside (1,4) vendu sous la dénomination "APG 300" par HENKEL à 50 g % en matière active (MA) 15 g M.A.
- N-(dodécyloxycarbonyl)-N-(2-hydroxy-3-hexadecyl oxypropyl)-éthanolamine (composé de l'exemple 2) 2 g
- conservateurs, parfum qs
- eau qsp 100 g

Ce shampooing présente l'aspect d'un liquide limpide. Il possède un bon pouvoir moussant et apporte de la douceur et de la discipline aux cheveux séchés.

### Exemple 6 : Après-shampooing

- Chlorure de béhényl triméthyl ammonium à 80 % en poids dans un mélange eau/isopropanol (15/85) vendu sous la dénomination "CATINAL DC 80" par TOHO 2 g M.A.
- N-(2-éthyl-hexyloxycarbonyl)-N-(2-hydroxy-3-hexadécyloxypropyl)-éthanolamine (composé de l'exemple 3) 0,5 g
- conservateurs, parfum qs
- Eau qsp 100 g
pH spontané 7,2

Cet après-shampooing présente l'aspect d'un liquide opalescent. Appliqué sur des cheveux, il améliore le démêlage des cheveux mouillés ainsi que la douceur et la discipline aux cheveux séchés.

### Exemple 7 : Crème de jour hydratante

- N-(hexadécyloxycarbonyl)-N-(2-hydroxy-3-hexadécyl oxypropyl)-éthanolamine (composé de l'exemple 1) 0,5 g
- Stéarate de glycéryle 2,5 g
- Huile minérale 6,2 g
- Myristate d'isopropyle 3 g
- Alcool cétylique 7 g
- PEG 50-stéarate 2,5 g
- Conservateur 0,3 g
- Eau qsp 100 g

On obtient une crème de jour se présentant sous forme d'une émulsion permettant de bien couvrir et protéger la peau, particulièrement adaptée pour les peaux normales et sèches et qui se révèle hydratante pour la peau.

### Exemple 8: Base de maquillage du visage hydratante

- Mélange d'octanoate de cétéaryle et de myristate d'isopropyle 2g
- N-(dodécyloxycarbonyl)-N-(2-hydroxy-3-hexadecyloxypropyl)-éthanolamine (composé de l'exemple 2) 0,5 g
- Huile minérale 7,5 g
- Mélange de stéarate de glycéryle et de PEG-100 stéarate 2 g
- Acide stéarique 1,4 g
- Alcool cétylique 0,1 g
- Hexyldécanol 1 g
- Glycérine 3 g
- Triéthanolamine 1,05 g
- Carbomer vendu sous la marque CARBOPOL 0,35 g
- Conservateur qs
- Eau qsp 100 g

### Exemple 9 : Crème de nuit hydratante pour la peau

- Mélange d'octanoate de cétéaryle et de myristate d'isopropyle 2 g
- N-(2-éthyl-hexyloxycarbonyl)-N-(2-hydroxy-3-hexadécyl oxypropyl)-éthanolamine (composé de l'exemple 3) 0,5 g
- Glycérine 10 g
- Gomme de cellulose 0,5 g
- Polyéthylène 0,5 g
- Sulfate de Magnésium 0,65 g
- Cyclométhicone 15,36 g
- Mélange d'isostéarate de polyglycéryle4, de cétyl diméthicone copolyol et de laurate d'hexyle 4 g
- Conservateur qs
- Eau qsp 100 g

On obtient une crème de nuit se présentant sous forme d'une émulsion permettant de bien couvrir et protéger la peau, particulièrement adaptée pour les peaux normales et sèches.

## Revendications

1. Composé répondant à la formule suivante : 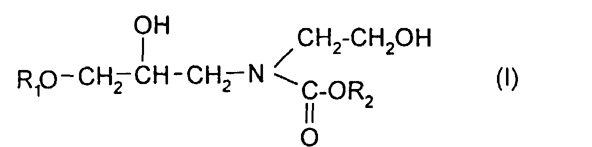 dans laquelle R₁ et R₂, identiques ou différents, représentent un radical alcoyle ayant de 8 à 26 atomes carbone, linéaire ou ramifié, saturé ou insaturé et éventuellement hydroxylé

2. Composé choisi dans le groupe constitué par la N-(hexadécyloxycarbonyl)-N-(2-hydroxy-3-hexadécyloxypropyl)-éthanolamine; la N-(dodécyloxycarbonyl)-N-(2-hydroxy-3-hexadécyloxypropyl)-éthanolamine; la N-(2-éthyl-hexyloxycarbonyl)-N-(2-hydroxy-3-hexadécyloxypropyl)-éthanolamine.

3. Procédé de préparation des composés de formule (I) selon la revendication 1 ou 2, caractérisé par le fait qu'il consiste à faire réagir, en présence d'un solvant organique, un composé de formule (II) suivante : 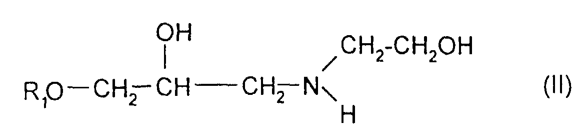 dans laquelle R₁ a les mêmes significations indiquées dans la revendication 1, avec un composé de formule (III) suivante : 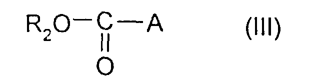 dans laquelle :
- A est un groupe activant choisi parmi un atome d'halogène et un azolide ;
- R₂ a les mêmes significations indiquées dans la revendication 1; puis à ajouter au milieu réactionnel obtenu une base organique.

4. Utilisation des composés de formule (I) selon la revendication 1 ou 2 dans et pour la préparation de compositions cosmétiques ou dermatologiques.

5. Composition cosmétique ou dermatologique contenant dans un véhicule cosmétiquement acceptable ou dermatologiquement acceptable au moins un composé de formule (I) suivante : 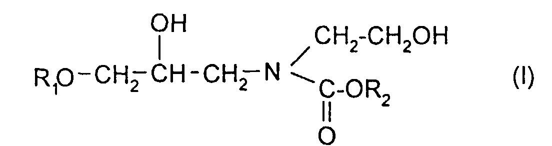 dans laquelle R₁ et R₂, identiques ou différents, représentent un radical alcoyle ayant de 8 à 26 atomes carbone, linéaire ou ramifié, saturé ou insaturé et éventuellement hydroxylé

6. Composition selon la revendication 5, où le composé de formule (I) est choisi dans le groupe constitué par la N-(hexadécyloxycarbonyl)-N-(2-hydroxy-3-hexadécyloxypropyl)-éthanolamine ; la N-(dodécyloxycarbonyl)-N-(2-hydroxy-3-hexadécyloxypropyl)-éthanolamine ; la N-(2-éthyl-hexyloxycarbonyl)-N-(2-hyd roxy-3-hexadécyloxypropyl)-éthanolamine.

7. Composition selon la revendication 5 ou 6, caractérisée par le fait qu'elle comprend de 0,001 à 15% en poids de composé de formule (I) par rapport au poids total de la composition.

8. Composition selon l'une quelconque des revendications 5 à 7, caractérisée par le fait qu'elle se présente sous forme de lotion aqueuse ou hydroalcoolique, monophasique ou polyphasique, de gel monophasique ou polyphasique, d'émulsion, de crème, de dispersion vésiculaire de lipides ioniques ou non ioniques, lesdites vésicules pouvant alors servir en tant qu'agent d'encapsulation pour des ingrédients actifs lipophiles ou hydrophiles, de mousse, de spray.

9. Composition selon l'une quelconque des revendications 5 à 8, caractérisée par le fait qu'elle peut contenir en plus des additifs cosmétiques conventionnels choisis parmi les corps gras, les solvants organiques, les silicones, les épaississants, les adoucissants, les tensio-actifs, les polymères anioniques, cationiques, non-ioniques ou amphotères, les agents anti-mousses, les agents conditionneurs du cheveu tels que des protéines, des vitamines, les agents traitants (agents antichute, antipelliculaires), les colorants, les parfums, les conservateurs, les agents propulseurs.

10. Utilisation d'au moins un composé de formule (I) tel que défini selon l'une quelconque des revendications 1 et 2, comme agent hydratant, dans et pour la préparation de compositions cosmétiques ou dermatologiques pour le soin de la peau.

11. Utilisation d'au moins un composé de formule (I) tel que défini selon l'une quelconque des revendications 1 et 2, comme agent de conditionnement des cheveux dans et pour la préparation de compositions capillaires.

## Patentansprüche

1. Verbindung der folgenden Formel 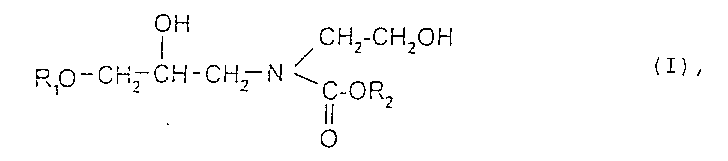 in der R₁ und R₂, die gleich oder verschieden sind, eine geradkettige oder verzweigte, gesättigte oder ungesattigte und gegebenenfalls hydroxygruppenhaltige Alkygruppe mit 8 bis 26 Kohlenstoffatomen darstellen.

2. Verbindung, die unter N-(Hexadecylcarbonyl)-N-(2-hydroxy-3-hexadecyloxypropyl)-ethanolamin, N-(Dodecyloxycarbonyl)-N-(2-hydroxy-3-hexadecyloxypropyl)-ethanolamin, N-(2-Ethylhexyloxycarbonyl)-N-(2-hydroxy-3-hexadecyloxypropyl)-ethanolamin ausgewählt ist.

3. Verfahren zur Herstellung der Verbindungen den Formel (I) nach Anspruch 1 oder 2, dadurch gekennzeichnet, daß in Gegenwart eines organischen Lösungsmittels eine Verbindung der folgenden Formel (II) 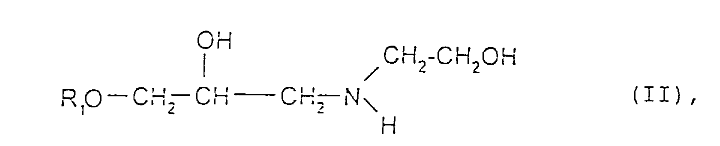 in der R₁ dasselbe wie in Anspruch 1 bedeutet, mit einer Verbindung der folgenden Formel (III) 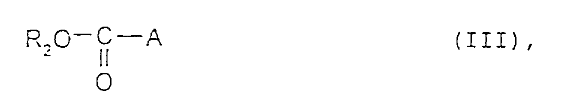 in der
- A₁ eine aktivierende Gruppe ist, die unter Halogenatomen und Azoliden ausgewählt wird,
- R₂ dasselbe wie in Anspruch 1 bedeutet,
umgesetzt wird, wonach zu dem erhaltenen Reaktionsmedium eine organische Base gegeben wird.

4. Verwendung der Verbindungen der Formel (I) nach Anspruch 1 oder 2 in oder für die Herstellung von kosmetischen oder dermatologischen zusammenstezungen.

5. Kosmetische oder dermatologische zusammensetzung, die in einem kosmetisch oder dermatologisch akzeptablen Vehikel mindestens eine Verbindung der folgenden Formel (I) 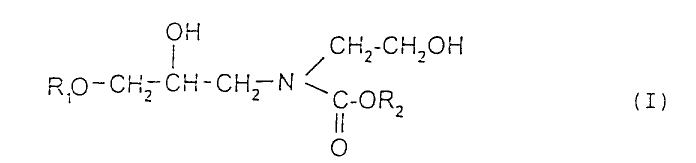 enthält, in der R₁ und R₂, die gleich oder verschieden sind, eine geradkettige oder verzweigte, gesättigte oder ungesättigte und gegebenfalls hydroxygruppenhaltige alkylgruppe mit 8 bis 26 Kohlenstoffatomen darstellen.

6. Zusammensetzung nach Anspruch 5, wobei die Verbindung der Formel (I) unter N-(Hexadecylcarbonyl)-N-(2-hydroxy-3-hexadecyloxypropyl)-ethanolamin, N-(Dodecyloxy-carbonyl)-N-(2-hydroxy-3-hexadecyloxypropyl)-ethanol-amin, N-(2-Ethylhexyloxycarbonyl)-N-(2-hydroxy-3-hexadecyloxypropyl)-ethanolamin ausgewählt ist.

7. Zusammensetzung nach Anspruch 5 oder 6, dadurch gekennzeichnet, daß sie 0,001 bis 15 Gew.-% Verbindung der Formel (I), bezogen auf das Gesamtgewicht der Zusammensetzung, enthält.

8. Zusammensetzung nach einem der Ansprüche 5 bis 7, dadurch gekennzeichnet, daß sie in Form einer einphasigen oder mehrphasigen, wäßrigen oder wäßrig-alkoholischen Lotion, eines einphasigen oder mehrphasigen Gels, einer Emulsion, einer Creme, einer Vesikeldispersion ionischer oder nichtionischer Lipide, wobei die Vesikel auch als Mittel zur Einkapselung von lipophilen oder hydrophilen Wirkstoffen dienen können, in Form eines Schaums, eines Sprays, vorliegt.

9. Zusammensetzung nach einem der Ansprüche 5 bis 8, dadurch gekennzeichnet daß sie außerdem herkömmliche kosmetische Zusätze enthalten kann, die unter Fettsubstanzen, organischen Lösungsmitteln, Siliconen, Verdikkungsmitteln, reizlindernden Mitteln, grenzflächenaktiven Stoffen, anionischen, kationischen, nichtionischen und amphoteren Polymeren, Antischaummitteln, Haarkonditioniermitteln, wie z.B. Proteinen, Vitaminen, Behandlungsmitteln (Mittel gegen Haarausfall, Antischuppenmittel), Färbemitteln, Parfums, Konservierungsmitteln, Treibmitteln ausgewählt sind.

10. Verwendung mindestens einer Verbindung der Formel (I), die wie in einem der Ansprüche 1 und 2 definiert ist, als Hydratisierungsmittel in und für die Herstellung von kosmetischen oder dermatologischen Zusammensetzungen für die hautpflege.

11. Verwendung mindestens einer Verbindung der Formel (I), die wie in einem der Ansprüche 1 und 2 definiert ist, als Konditioniermittel für die Haare in und für die Herstellung von Zusammensetzungen für die Haarbehandlung.

## Claims

1. Compound corresponding to the following formula: 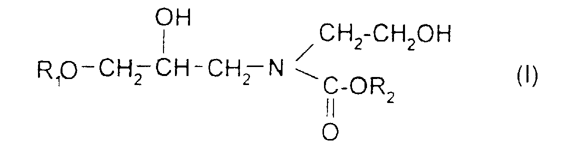 in which R₁ and R₂, which may be identical or different, represent a linear or branched, saturated or unsaturated and optionally hydroxylated alkyl radical having from 8 to 26 carbon atoms.

2. Compound chosen from the group consisting of N-(hexadecyloxycarbonyl)-N-(2-hydroxy-3-hexadecyloxypropyl)ethanolamine; N-(dodecyloxycarbonyl)-N-(2-hydroxy-3-hexadecyloxypropyl)ethanol-amine; N-(2-ethylhexyloxy-carbonyl)-N-(2-hydroxy-3-hexadecyloxypropyl)ethanolamine.

3. Process for the preparation of the compounds of formula (I) according to Claim 1 or 2, characterized in that it consists in reacting, in the presence of an organic solvent, a compound of formula (II) below: 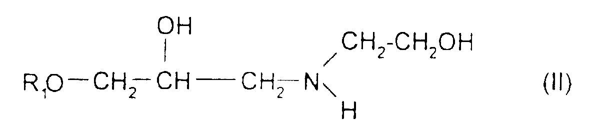 in which R₁ has the same meanings indicated in Claim 1, with a compound of formula (III) below: 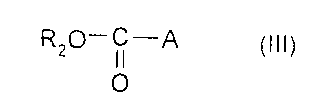 in which:
- A is an activating group chosen from a halogen atom and an azolide;
- R₂ has the same meanings indicated in Claim 1; next, in adding an organic base to the reaction medium obtained.

4. Use of the compounds of formula (I) according to Claim 1 or 2 in and for the preparation of cosmetic or dermatological compositions.

5. Cosmetic or dermatological composition containing, in a cosmetically acceptable or dermatologically acceptable vehicle, at least one compound of formula (I) below: 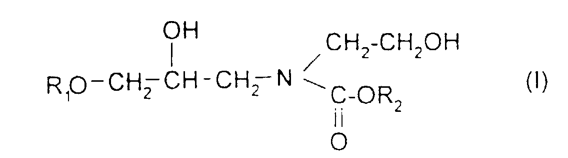 in which R₁ and R₂, which may be identical or different, represent a linear or branched, saturated or unsaturated and optionally hydroxylated alkyl radical having from 8 to 26 carbon atoms.

6. Composition according to Claim 5, where the compound of formula (I) is chosen from the group consisting of N-(hexadecyloxycarbonyl)-N-(2-hydroxy-3-hexadecyloxypropyl)ethanolamine; N-(dodecyloxycarbonyl)-N-(2-hydroxy-3-hexadecyloxypropyl)ethanolamine; N-(2-ethylhexyloxycarbonyl)-N-(2-hydroxy-3-hexadecyloxypropyl)ethanolamine.

7. Composition according to Claim 5 or 6, characterized in that it comprises from 0.001 to 15% by weight of compound of formula (I) relative to the total weight of the composition.

8. Composition according to any one of Claims 5 to 7, characterized in that it is in the form of a monophasic or polyphasic aqueous or aqueous-alcoholic lotion, a monophasic or polyphasic gel, an emulsion, a cream or a vesicle dispersion of ionic or nonionic lipids, it being possible for the said vesicles then to serve as encapsulating agents for lipophilic or hydrophilic active ingredients, a mousse or a spray.

9. Composition according to any one of Claims 5 to 8, characterized in that it may also contain conventional cosmetic additives chosen from fatty substances, organic solvents, silicones, thickeners, softeners, surfactants, anionic, cationic, nonionic or amphoteric polymers, antifoaming agents, hair conditioners such as proteins, vitamins, treating agents (agents for preventing hair loss or antidandruff agents), dyes, fragrances, preserving agents and propellants.

10. Use of at least one compound of formula (I) as defined according to either of Claims 1 and 2, as a moisturizing agent in, and for the preparation of, cosmetic or dermatological skincare compositions.

11. Use of at least one compound of formula (I) as defined according to either of Claims 1 and 2, as a hair conditioner in, and for the preparation of, hair compositions.
